# EUROPÄISCHE PATENTSCHRIFT

(11) **EP 1 697 318 B1**
(45) Veröffentlichungstag und Bekanntmachung des Hinweises auf die Patenterteilung: **27.08.2008**
(21) Anmeldenummer: 04820404.4
(22) Anmeldetag: 26.11.2004
(51) Int. Cl.: C07D 207/12, C07D 401/12, A61K 31/40, A61P 35/00, A61P 7/02, A61P 9/10, A61P 25/00

(54) **PROLINYLARYLACETAMIDE**
PROLINYLARYLACETAMIDES
PROLINYLARYLACETAMIDE

(30) Priorität: 16.12.2003 DE 10358814
(43) Veröffentlichungstag der Anmeldung: 06.09.2006
(73) Patentinhaber: Merck Patent GmbH, 64293 Darmstadt (DE)
(72) Erfinder: MEDERSKI, Werner, 64673 Zwingenberg (DE); TSAKLAKIDIS, Christos, 69469 Weinheim (DE); DORSCH, Dieter, 64372 Ober-Ramstadt (DE); CEZANNE, Bertram, 64546 Mörfelden-Walldorf (DE); GLEITZ, Johannes, 64289 Darmstadt (DE)
(86) Internationale Anmeldenummer: PCT/EP2004/013509
(87) Internationale Veröffentlichungsnummer: WO 2005/058817

(56) Entgegenhaltungen:
- WO-A-03/045912
- WO-A-20/04087646
- WO-A-20/04087696

## Beschreibung

Die Erfindung betrifft Verbindungen ausgewählt aus der Gruppe
(2R,4R)-4-Hydroxy-pyrrolidin-1,2-dicarbonsäure-1-[(4-chlorphenyl)-amid]-2-({4-[(2-dimethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(N-methyl,N-butyl-amino)-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-(morpholin-4-yl)-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(4-hydroxy-piperidin-1-yl)-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-(2,6-dimethyl-morpholin-4-yl)-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(3-cyclohexylmethyl-piperidin-1-yl)-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-diethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(*N*-methyl,*N*-ethyl-amino)-ethanoyl)-methylaminol-phenyl}-amid),
(2R,4R)-4-Hydroxy-pyrrolidin-1,2-dicarbonsäure-1-[(4-chlorphenyl)-amid]-2-({4-[(2-(2-methyl-imidazol-1-yl)-ethanoyl)-methylamino]-phenyl}-amid),
(2R,4R)-4-Hydroxy-pyrrolidin-1,2-dicarbonsäure-1-[(4-ethinylphenyl)-amid]-2-({4-[(2-dimethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({2-fluor-4-[(2-dimethylamino-ethanoyl)-methylamino]-phenyl}-amid),
(2R,4R)-4-Hydroxy-pyrrolidin-1,2-dicarbonsäure-1-[(4-chlorphenyl)-amid]-2-({5-[(2-dimethylamino-ethanoyl)-methyl-amino]-pyridin-2-yl}-amid),
(2R,4R)-4-Hydroxy-pyrrolidin-1,2-dicarbonsäure-1-[(4-chlorphenyl)-amid]-2-({4-[(2-acetoxy-ethanoyl)-methyl-amino]-phenyl}amid),
(2R,4R)-2-[({[4-({1-[1-(4-Chlor-phenylcarbamoyl)-4-hydroxypyrrolidin-2-yl]-methanoyl}-amino)-phenyl]-methyl-carbamoyl}methyl)-amino]-4-methyl-pentansäure-methylester,
(2R,4R)-4-Hydroxy-pyrrolidin-1,2-dicarbonsäure-1-[(4-chlorphenyl)-amid]-2-({4-[(2-ethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-chlor-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-cyclohexylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-methylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-isopropylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-tert.-butylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-cyclopentylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-cyclopropylmethylamino-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-hydroxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-methoxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-ethoxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-propoxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-butoxy-ethanoyl)-methyl-amino]-phenyl}amid),
(2R,4R)-4-Hydroxy-pyrrolidin-1,2-dicarbonsäure-1-[(4-ethinylphenyl)-amid]-2-({4-[(2-methoxy-ethanoyl)-methyl-amino]-phenyl}amid),
(2R,4R)-4-Hydroxy-pyrrolidin-1,2-dicarbonsäure-1-[(4-chlorphenyl)-amid]-2-({2-fluor-4-[(2-methoxy-ethanoyl)-methyl-amino]-phenyl}-amid),
(2R,4R)-4-Hydroxy-pyrrolidin-1,2-dicarbonsäure-1-[(4-chlorphenyl)-amid]-2-({5-[(2-methoxy-ethanoyl)-methyl-amino]-pyridin-2-yl}-amid),
sowie ihre pharmazeutisch verwendbaren Solvate, Salze und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die erfingungsgemäβen Verbindungen und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen können weiterhin Inhibitoren der Gerinnungsfaktoren Faktor VIIa, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.
Andere Carbonsäureamidderivate sind aus WO 02/48099 und WO 02/57236 bekannt, andere Pyrrolidinderivate sind in WO 02/100830 beschrieben.
Weitere heterocyclische Derivate kennt man aus der WO 03/045912. Pyrrolidinderivate als Inhibitoren des Endothelin-Converting-Enzyms sind aus der WO 02/06222 bekannt.
Pyrrolidinderivate als Cholecystokinin- und Gastrin-Inhibitoren sind in der US 5,340, 801 beschrieben. Andere Pyrrolidinderivate kennt man aus WO 01/044192.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor Vlla, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren. Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.
Die erfindungsgemäßen Verbindungen sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.
Die Inhibierung des Faktors Vlla durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor Vlla wird z.B. von H. F. Ronning et al. in Thrombosis Research 1996, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor lXa kann daher auf andere Weise verhindern, daß Faktor Xa gebildet wird.
Die inhibierung von Faktor lXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor Vlla und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. lnvest. 104: 1213-1221 (1999);
B.M. Mueller et al. in J. Clin. lnvest. 101: 1372-1378 (1998);
M.E. Bromberg et al. in Thromb. Haemost. 1999; 82: 88-92
Die Verbindungen können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo,* oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).
Darüberhinaus können sie zur Behandlung von Tinnitus verwendet werden. Die Verwendung von Antikoagulantien bei der Tinnitustherapie ist von R. Mora et al. in International Tinnitus Journal (2003), 9(2), 109-111 beschrieben.

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Die erfingungsgemäβen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäβen Verbindungen umsetzt.

Die erfindungsgemäβen Verbingunger können vorzugsweise erhalten werden, indem man vorzugsweise unter Ullmann-Reaktionsbedingungen (Cul, K₂CO₃, DMSO, 130°) oder, besonders bevorzugt unter Bedingungen einer *Buchwald*-Amidierung (J. Am. Chem. Soc. 2002, 121, 7421) umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, N,N'-Dimethylendiamin, Pyridin oder Chinolin ist geeignet. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°, besonders bevorzugt zwischen 60 und 90°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäβen Verbindungen werden größtenteils konventionell hergestellt. Sofern die Verbindung eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der erfindungsgemäβen Verbindungen zählen ebenfalls dazu. Bei bestimmten erfindungsgemäβen Verbindungen lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der erfindungsgemäβen Verbindungen, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer erfindungsgemäβen Verbindungen werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine erfindungsgemäβe Verbindung in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanoll Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäβe Verbindung und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Ol-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden.
Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate-aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs-oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäβen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäβen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden.
Die erfindungsgemäβen Verbindungen und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen verwendet werden.

Gegenstand der Erfindung sind ferner Arzneimittel enthaltend mindestens eine erfindungsgemäβe Verbindung und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäβen Verbindung und/oder ihrer pharmazeutisch verwendbaren Derivate, Solvate und Stereoisomere, einschließlich deren Mischungen in allen Verhältnissen, und
(b) einer wirksamen Menge eines weiteren Arzneimiftelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäβen Verbindung und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

Gegenstand der Erfindung ist ferner die Verwendung von erfindungsgemäβen Verbindungen und/oder ihrer pharmazeutisch verwendbaren Derivate, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:
Ethylacetat/Methanol 9:1.
Massenspektrometrie (MS): El (Elektronenstoß-lonisation) M⁺
ESl (Electrospray lonization) (M+H)⁺ (wenn nichts anderes angegeben)

### Beispiel 1

### (2R,4R)-4-Hydroxy-pyrrolidin-1,2-dicarbonsäure-1-[(4-chlor-phenyl)-amid]-2-({4-[(2-dimethylamino-ethanoyl)-methyl-amino]-phenyl}-amid) ("A1")

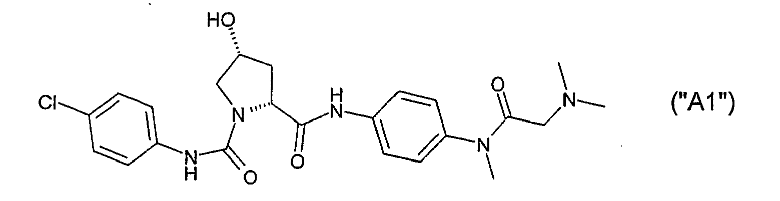

100 mg (0.351 mmol) (2R,4R)-1-(4-Chlorphenyl-carbamoyl)-4-hydroxy-prolin (1) und 72.75 mg (0.351 mmol) N-(4-Aminophenyl)-2-dimethylamino-N-methyl-acetamid (beschrieben in US 2436115, 1945) werden in 1 mL DMF gelöst, mit 62.29 mg (0.351 mmol) N-(3-Dimethylaminopropyl)-N'-ethyl-carbodiimidhydrochlorid versetzt und 24 h bei RT gerührt. Nach üblicher Aufarbeitung erhält man 35 mg (21 %) "A1"; (M+H)⁺475; F. 95°.

Analog werden nachstehende Verbindungen erhalten
(2R,4R)-4-Hydroxy-pyrrolidin-1,2-dicarbonsäure-1-[(4-chlor-phenyl)-amid]-2-({4-[(2-(*N*-methyl,*N*-butyl-amino)-ethanoyl)-methyl-amino]-phenyl}-amid), F. 98°;
(2R,4R)-4-Hydroxy-pyrrolidin-1,2-dicarbonsäure-1-[(4-chlor-phenyl)-amid]-2-({4-[(2-(morpholin-4-yl)-ethanoyl)-methyl-amino]-phenyl}-amid) ("A1-1"), F. 86°;
(2R,4R)-4-Hydroxy-pyrrolidin-1,2-dicarbonsäure-1-[(4-chlor-phenyl)-amid]-2-({4-[(2-(4-hydroxy-piperidin-1-yl)-ethanoyl)-methyl-amino]-phenyl}-amid) ("A1-2"), F. 78°;
(2R,4R)-4-Hydroxy-pyrrolidin-1,2-dicarbonsäure-1-[(4-chlor-phenyl)-amid]-2-({4-[(2-(2,6-dimethyl-morpholin-4-yl)-ethanoyl)-methyl-amino]-phenyl}-amid), F. 137°;
(2R,4R)-4-Hydroxy-pyrrolidin-1,2-dicarbonsäure-1-[(4-chlor-phenyl)-amid]-2-({4-[(2-(3-cyclohexylmethyl-piperidin-1-yl)-ethanoyl)-methyl-amino]-phenyl}-amid), F. 104°;
(2R,4R)-4-Hydroxy-pyrrolidin-1,2-dicarbonsäure-1-[(4-chlor-phenyl)-amid]-2-({4-[(2-diethylamino-ethanoyl)-methyl-amino]-phenyl}-amid), F. 86°;
(2R,4R)-4-Hydroxy-pyrrolidin-1,2-dicarbonsäure-1-[(4-chlor-phenyl)-amid]-2-({4-[(2-(*N*-methyl,*N*-ethyl-amino)-ethanoyl)-methyl-amino]-phenyl}-amid), F. 113°;
(2R,4R)-4-Hyd roxy-pyrrolidin-1,2-dicarbonsäu re-1-[(4-chlor-phenyl)-amid]-2-({4-[(2-(2-methyl-imidazol-1-yl)-ethanoyl)-methyl-amino]-phenyl}-amid), F. 171°;
(2R,4R)-4-Hydroxy-pyrrolidin-1,2-dicarbonsäure-1-[(4-ethinyl-phenyl)-amid]-2-({4-[(2-dimethylamino-ethanoyl)-methyl-amino]-phenyl}-amid),
(2R,4R)-4-Hydroxy-pyrrolidin-1,2-dicarbonsäure-1-[(4-chlor-phenyl)-amid]-2-({2-fluor-4-[(2-dimethylamino-ethanoyl)-methyl-amino]-phenyl}-amid),
(2R,4R)-4-Hydroxy-pyrrolidin-1,2-dicarbonsäure-1-[(4-chlor-phenyl)-amid]-2-({5-[(2-dimethylamino-ethanoyl)-methyl-amino]-pyridin-2-yl}-amid),

### Beispiel 2

### (2R,4R)-4-Hydroxy-pyrrolidin-1,2-dicarbonsäure-1-[(4-chlor-phenyl)-amid]-2-({4-[(2-acetoxy-ethanoyl)-methyl-amino]-pheny/}-amid) ("A2")

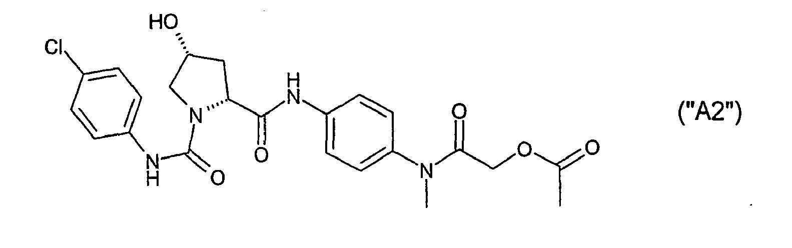

2.1 1.146 g (4.025 mmol) 1 werden in 10 mL THF suspendiert, mit 0.995 g (4.025 mmol) EEDQ (= Ethyl-2-ethoxy-1,2-dihydrochinolin-1-carboxylat versetzt und 30 min bei RT gerührt. Nach Zugabe von 0.8 g (4.027 mmol) 4-Methylamino-anilin (beschrieben in J. Org. Chem. 26, 1961, 1394) wird die Reaktionsmischung weitere 18 h bei RT gerührt. Nach üblicher Aufarbeitung erhält man so 300 mg (2R,4R)-4-Hydroxy-pyrrolidin-1,2-dicarbonsäure-1-[(4-chlor-phenyl)-amid]-2-({4-(N-methyl-amino)-phenyl}-amid) (2), (M+H)⁺ 390.

### 2.2 240 mg (0.617 mmol) (2) werden in 2 mL DCM gelöst und

nacheinander mit 82.92 µL (0.771 mmol) Acetoxyessigsäurechlorid, 62.23 µL (0.771 mmol) Pyridin und 0.977 mg (0.008 mmol) DMAP (= 4-(Dimethylamino)-pyridin) versetzt. Man rührt anschliessend 18 h bei RT und arbeitet wie üblich auf. So erhält man 125 mg (41%) "A2", MS = 490 (M+H)⁺.

Analog erhält man die nachstehende Verbindung
(2R,4R)-2-[({[4-({1-[1-(4-Chlor-phenylcarbamoyl)-4-hydroxy-pyrrolidin-2-yl]-methanoyl}-amino)-phenyl]-methyl-carbamoyl}-methyl)-amino]-4-methyl-pentansäure-methylester, F. 86°

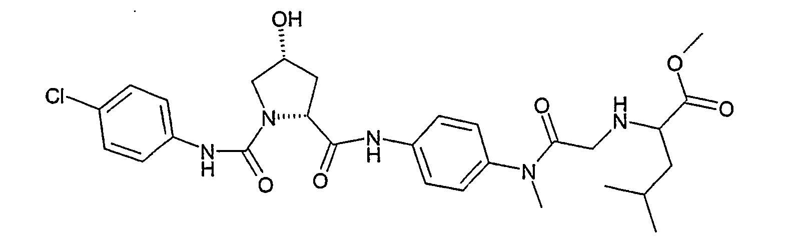

### Beispiel 3

### (2R,4R)-4-Hydroxy-pyrrolidin-1,2-dicarbonsäure-1-[(4-chlor-phenyl)-amid]-2-({4-[(2-ethylamino-ethanoyl)-methyl-amino]-phenyl}-amid) ("A3")

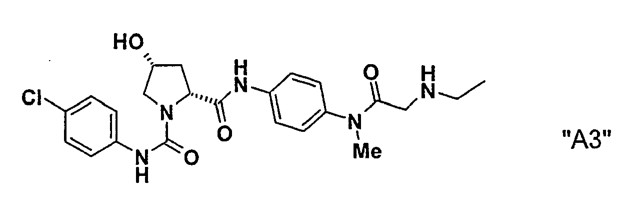

### 3.1 Herstellung von N-(4-Aminophenyl)-2-chlor-N-methylacetamid (4)

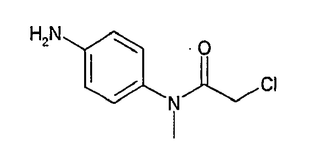

1.0g (4.374 mmol) 2-Chlor-*N*-methyl-*N*-(4-nitro-phenyl)-acetamid **3** (beschrieben in Biochem. J. 55, 1953, 839) werden in 25 mL THF gelöst und mit 0.5 g Pt-C (5%)-55.9% wasserfeucht bei RT hydriert. Nach üblicher Aufarbeitung erhält man **4**.

3.2 1.146 g (4.025 mmol) **1** werden in 10 mL THF suspendiert, mit 0.995 g (4.025 mmol) EEDQ (= Ethyl-2-ethoxy-1,2-dihydrochinolin-1-carboxylat versetzt und 30 min bei RT gerührt. Nach Zugabe von 0.8 g (4.027 mmol) **4** wird die Reaktionsmischung weitere 18 h bei RT gerührt. Nach üblicher Aufarbeitung erhält man so 750 mg (40%) *(2R,4R)-4-Hydroxy-pyrrolidin-1,2-dicarbonsäure-1-[(4-chlor-phenyl)-amid]-2-({4-[(2-chlor-ethanoyl)-methyl-amino]-phenyl}-amid)* ("A3a"); (M+H)⁺ 466.

3.3 250 mg (0.537 mmol) "A3a" und 406 µL (0.806 mmol) Ethylamin (2M in THF) werden in 2 mL Acetonitril gelöst, mit 85.4 mg (0.806) wasserfreiem Natriumcarbonat versetzt und 5 h bei 60°C gerührt. Nach üblicher Aufarbeitung erhält man so 81 mg (32%) "A3"; (M+H)⁺475; F. 121 °.

Analog werden nachstehende Verbindungen erhalten
(2R,4R)-4-Hydroxy-pyrrolidin-1,2-dicarbonsäure-1-[(4-chlor-phenyl)-amid]-2-({4-[(2-cyclohexylamino-ethanoyl)-methyl-amino]-phenyl}-amid), F. 141 °;
(2R,4R)-4-Hydroxy-pyrrolidin-1,2-dicarbonsäure-1-[(4-chlor-phenyl)-amid]-2-({4-[(2-methylamino-ethanoyl)-methyl-amino]-phenyl}-amid), F. 145°;
(2R,4R)-4-Hydroxy-pyrrolidin-1,2-dicarbonsäure-1-[(4-chlor-phenyl)-amid]-2-({4-[(2-isopropylamino-ethanoyl)-methyl-amino]-phenyl}-amid), F. 123.5°;
(2R,4R)-4-Hydroxy-pyrrolidin-1,2-dicarbonsäure-1-[(4-chlor-phenyl)-amid]-2-({4-[(2-tert.-butylamino-ethanoyl)-methyl-amino]-phenyl}-amid), F. 137°;
(2R,4R)-4-Hydroxy-pyrrolidin-1,2-dicarbonsäure-1-[(4-chlor-phenyl)-amid]-2-({4-[(2-cyclopentylamino-ethanoyl)-methyl-amino]-phenyl}-amid), F. 130°;
(2R,4R)-4-Hydroxy-pyrrolidin-1,2-dicarbonsäure-1-[(4-chlor-phenyl)-amid]-2-({4-[(2-cyclopropylmethylamino-ethanoyl)-methyl-amino]-phenyl}-amid), F. 126°;

Aus (2R,4R)-4-Hydroxy-pyrrolidin-1,2-dicarbonsäure-1-[(4-chlor-phenyl)-amid]-2-({4-[(2-chlor-ethanoyl)-methyl-amino]-phenyl}-amid) ("A3a") erhält man durch Chlorsubstitution die Verbindung
(2R,4R)-4-Hydroxy-pyrrolidin-1,2-dicarbonsäure-1-[(4-chlor-phenyl)-amid]-2-({4-[(2-hydroxy-ethanoyl)-methyl-amino]-phenyl}-amid).

### Beispiel 4

### (2R,4R)-4-Hydroxy-pyrrolidin-1,2-dicarbonsäure-1-[(4-chlor-phenyl)-amid]-2-({4-[(2-methoxy-ethanoyl)-methyl-amino]-phenyl}-amid) ("A4")

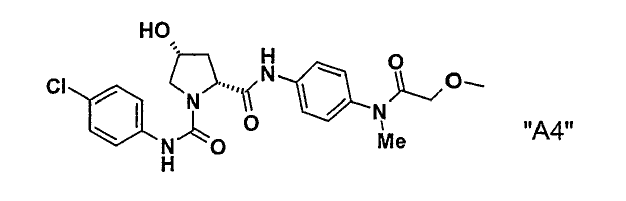

Unter Argon werden in einen Kolben 0.05 mmol Cul (5mol%), 1.5 mmol 2-Methoxy-acetamid und 2.03 mmol K₃PO₄ gegeben. Nach Zugabe von 1.0 mL Toluol werden 0.1 mmol (10 mol%) N,N'-Dimethylendiamin und 1.0 mmol (2R,4R)-4-Hydroxy-pyrrolidin-1,2-dicarbonsäure-1-[(4-chlor-phenyl)-amid]-2-({4-iod-phenyl}-amid) (5), (M+H)⁺487 [Herstellung analog Beispiel 3.2] hinzugefügt und 12 h bei 80°C gerührt. Nach dem Abkühlen wird wie üblich aufgearbeitet und man erhält so "A4"; (M+H)⁺ 462; F. 84°.

Analog erhält man die nachstehenden Verbindungen
(2R,4R)-4-Hydroxy-pyrrolidin-1,2-dicarbonsäure-1-[(4-chlor-phenyl)-amid]-2-({4-[(2-ethoxy-ethanoyl)-methyl-amino]-phenyl}-amid), F. 89°;
(2R,4R)-4-Hydroxy-pyrrolidin-1,2-dicarbonsäure-1-[(4-chlorphenyl)-amid]-2-({4-[(2-propoxy-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-butoxy-ethanoyl)-methyl-amino]-phenyl}-amid),
(2R,4R)-4-Hydroxy-pyrrolidin-1,2-dicarbonsäure-1-[(4-ethinylphenyl)-amid]-2-({4-[(2-methoxy-ethanoyl)-methyl-amino]-phenyl}-amid),
(2R,4R)-4-Hydroxy-pyrrolidin-1,2-dicarbonsäure-1-[(4-chlorphenyl)-amid]-2-({2-fluor-4-[(2-methoxy-ethanoyl)-methyl-amino]-phenyl}-amid),
(2R,4R)-4-Hydroxy-pyrrolidin-1,2-dicarbonsäure-1-[(4-chlorphenyl)-amid]-2-({5-[(2-methoxy-ethanoyl)-methyl-amino]-pyridin-2-yl}amid).

### Pharmakologische Daten

### Affinität zu Rezeptoren

**Tabelle 1**

| Verbindung Nr. | FXa-IC₅₀ [nM] | TF/FVIIa-IC₅₀ [M] |
|---|---|---|
| "A1" | | 37.0 |
| "A1-1" | 54.0 | 100.0 |
| "A1-2" | 37.0 | 46.0 |
| "A2" | | |
| "A3" | 17.0 | 25.0 |

Die nachfolgenden Beispiele betreffen pharmazeutische Zubereitungen:

### Beispiel A: Injektionsgläser

Eine Lösung von 100 g eines erfindungsgemäβen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäβen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäβen Wirkstoffes , 9,38 g NaH₂PO₄ - 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäβen Wirkstoffes mit 99,5 Vaseline unter aseptischen Bedingungen.

### Beispiel E: Tabletten

Ein Gemisch von 1 kg Wirkstoff , 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff in 60 l zweifach destilliertem Wasser wird steril filtriert, in Ampullen abgefühlt, unter sterilen Bedingungen lyophilisiert und steril verschlossen. Jede Ampulle enthält 10 mg Wirkstoff.

## Patentansprüche

1. Verbindungen ausgewählt aus der Gruppe
(2R,4R)-4-Hydroxy-pyrrolidin-1,2-dicarbonsäure-1-[(4-chlorphenyl)-amid]-2-({4-[(2-dimethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(*N*-methyl,*N*-butyl-amino)-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-(morpholin-4-yl)-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(4-hydroxy-piperidin-1-yl)-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-(2,6-dimethyl-morpholin-4-yl)-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(3-cyclohexylmethyl-piperidin-1-yl)-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-diethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-(*N*-methyl,*N*-ethyl-amino)-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-(2-methyl-imidazol-1-yl)-ethanoyl)-methylamino]-phenyl}-amid),
(2R,4R)-4-Hydroxy-pyrrolidin-1,2-dicarbonsäure-1-[(4-ethinyl-phenyl)-amid]-2-({4-[(2-dimethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({2-fluor-4-[(2-dimethylamino-ethanoyl)-methylamino]-phenyl}-amid),
(2R,4R)-4-Hydroxy-pyrrolidin-1,2-dicarbonsäure-1-[(4-chlorphenyl)-amid]-2-({5-[(2-dimethylamino-ethanoyl)-methyl-amino]-pyridin-2-yl}-amid),
(2R,4R)-4-Hydroxy-pyrrolidin-1,2-dicarbonsäure-1-[(4-chlorphenyl)-amid]-2-({4-[(2-acetoxy-ethanoyl)-methyl-amino]-phenyl}-amid),
(2R,4R)-2-[({[4-({1-[1 -(4-Chlor-phenylcarbamoyl)-4-hydroxy-pyrrolidin-2-yl]-methanoyl}-amino)-phenyl]-methyl-carbamoyl}-methyl)-amino]-4-methyl-pentansäure-methylester,
(2R,4R)-4-Hydroxy-pyrrolidin-1,2-dicarbonsäure-1-[(4-chlorphenyl)-amid]-2-({4-[(2-ethylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-chlor-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-cyclohexylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-methylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-isopropylamino-ethanoyl)-methyl-amino]-phenyl}-amid),
(2R,4R)-4-Hydroxy-pyrrolidin-1,2-dicarbonsäure-1-[(4-chlorphenyl)-amid]-2-({4-[(2-tert.-butylamino-ethanoyl)-methyl-amino]phenyl}-amid),
(2R,4R)-4-Hydroxy-pyrrolidin-1,2-dicarbonsäure-1-[(4-chlorphenyl)-amid]-2-({4-[(2-cyclopentylamino-ethanoyl)-methyl-amino]phenyl}-amid),
(2R,4R)-4-Hydroxy-pyrrolidin-1,2-dicarbonsäure-1-[(4-chlorphenyl)-amid]-2-({4-[(2-cyclopropylmethylamino-ethanoyl)-methylamino]-phenyl}-amid),
(2R,4R)-4-Hydroxy-pyrrolidin-1,2-dicarbonsäure-1-[(4-chlorphenyl)-amid]-2-({4-[(2-hydroxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-methoxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-ethoxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-propoxy-ethanoyl)-methyl-amino]-phenyl}amid),
(2R,4R)-4-Hydroxy-pyrrolidin-1,2-dicarbonsäure-1-[(4-chlorphenyl)-amid]-2-({4-[(2-butoxy-ethanoyl)-methyl-amino]-phenyl}amid),
(2R,4R)-4-Hydroxy-pyrrolidin-1,2-dicarbonsäure-1-[(4-ethinylphenyl)-amid]-2-({4-[(2-methoxy-ethanoyl)-methyl-amino]-phenyl}amid),
(2R,4R)-4-Hydroxy-pyrrolidin-1,2-dicarbonsäure-1-[(4-chlorphenyl)-amid]-2-({2-fluor-4-[(2-methoxy-ethanoyl)-methyl-amino]-phenyl}-amid),
(2R,4R)-4-Hydroxy-pyrrolidin-1,2-dicarbonsäure-1-[(4-chlorphenyl)-amid]-2-({5-[(2-methoxy-ethanoyl)-methyl-amino]-pyridin-2-yl}-amid),
sowie ihre pharmazeutisch verwendbaren Solvate, Salze und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Solvate und Stereoisomere, einschließlich deren Mischungen in allen Verhältnissen, sowie gegebenenfalls Träger- und/oder Hilfsstoffe.

3. Arzneimittel enthaltend mindestens eine Verbindung nach Anspruch 1 und/oder ihre pharmazeutisch verwendbaren Solvate und Stereoisomere, einschließlich deren Mischungen in allen Verhältnissen, und mindestens einen weiteren Arzneimittelwirkstoff.

4. Verwendung von Verbindungen nach Anspruch 1 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

5. Set (Kit), bestehend aus getrennten Packungen von
(a) einer wirksamen Menge an einer Verbindung nach Anspruch 1 und/oder ihrer pharmazeutisch verwendbaren Solvate und Stereoisomere, einschließlich deren Mischungen in allen Verhältnissen,
und
(b) einer wirksamen Menge eines weiteren Arzneimittelswirkstoffs.

6. Verwendung von Verbindungen nach Anspruch 1 und/oder ihrer pharmazeutisch verwendbaren Solvate und Stereoisomere, einschließlich deren Mischungen in allen Verhältnissen, zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

## Claims

1. Compounds selected from the group
1-N-(4-chlorophenyl)-2-N-{4-[(2-dimethylaminoethanoyl)-methylamino]phenyl}-(2R,4R)-4-hydroxypyrrolidine-1,2-dicarboxamide,
1-N-(4-chlorophenyl)-2-N-{4-[(2-(*N*-methyl,*N*-butylamino)-ethanoyl)methylamino]phenyl}-(2R,4R)-4-hydroxypyrrolidine-1,2-dicarboxamide,
1-N-(4-chlorophenyl)-2-N-{4-[(2-(morpholin-4-yl)ethanoyl)methylamino]phenyl}-(2R,4R)-4-hydroxypyrrolidine-1,2-dicarboxamide,
1-N-(4-chlorophenyl)-2-N-{4-[(2-(4-hydroxypiperidin-1-yl)-ethanoyl)methylamino]phenyl}-(2R,4R)-4-hydroxypyrrolidine-1,2-dicarboxamide,
1-N-(4-chlorophenyl)-2-N-{4-[(2-(2,6-dimethylmorpholin-4-yl)-ethanoyl)methylamino]phenyl}-(2R,4R)-4-hydroxypyrrolidine-1,2-dicarboxamide,
1-N-(4-chlorophenyl)-2-N-{4-[(2-(3-cyclohexylmethylpiperidin-1-yl)ethanoyl)methylamino]phenyl}-(2R,4R)-4-hydroxypyrrolidine-1,2-dicarboxamide,
1-N-(4-chlorophenyl)-2-N-{4-[(2-diethylaminoethanoyl)methylamino]phenyl}-(2R,4R)-4-hydroxypyrrolidine-1,2-dicarboxamide,
1-N-(4-chlorophenyl)-2-N-{4-[(2-(*N*-methyl,*N*-ethylamino)-ethanoyl)methylamino]phenyl}-(2R,4R)-4-hydroxypyrrolidine-1,2-dicarboxamide,
1-N-(4-chlorophenyl)-2-N-{4-[(2-(2-methylimidazol-1-yl)-ethanoyl)methylamino]phenyl}-(2R,4R)-4-hydroxypyrrolidine-1,2-dicarboxamide,
1-N-(4-ethynylphenyl)-2-N-{4-[(2-dimethylaminoethanoyl)methylamino]phenyl}-(2R,4R)-4-hydroxypyrrolidine-1,2-dicarboxamide,
1-N-(4-chlorophenyl)-2-N-{2-fluoro-4-[(2-dimethylaminoethanoyl)methylamino]phenyl}-(2R,4R)-4-hydroxypyrrolidine-1,2-dicarboxamide,
1-N-(4-chlorophenyl)-2-N-{5-[(2-dimethylaminoethanoyl)methylamino]pyridin-2-yl}-(2R,4R)-4-hydroxypyrrolidine-1,2-dicarboxamide,
1-N-(4-chlorophenyl)-2-N-{4-[(2-acetoxyethanoyl)methylamino]-phenyl}-(2R,4R)-4-hydroxypyrrolidine-1,2-dicarboxamide,
methyl (2R,4R)-2-[({[4-({1-[1-(4-chlorophenylcarbamoyl)-4-hydroxypyrrolidin-2-yl]methanoyl}amino)phenyl]methylcarbamoyl}methyl)amino]-4-methylpentanoate,
1-N-(4-chlorophenyl)-2-N-{4-[(2-ethylaminoethanoyl)methylamino]phenyl}-(2R,4R)-4-hydroxypyrrolidine-1,2-dicarboxamide,
1-N-(4-chlorophenyl)-2-N-{4-[(2-chloroethanoyl)methylamino]-phenyl}-(2R,4R)-4-hydroxypyrrolidine-1,2-dicarboxamide,
1-N-(4-chlorophenyl)-2-N-{4-[(2-cyclohexylaminoethanoyl)-methylamino]phenyl}-(2R,4R)-4-hydroxypyrrolidine-1,2-dicarboxamide,
1-N-(4-chlorophenyl)-2-N-{4-[(2-methylaminoethanoyl)methylamino]phenyl}-(2R,4R)-4-hydroxypyrrolidine-1,2-dicarboxamide,
1-N-(4-chlorophenyl)-2-N-{4-[(2-isopropylaminoethanoyl)methylamino]phenyl}-(2R,4R)-4-hydroxypyrrolidine-1,2-dicarboxamide,
1-N-(4-chlorophenyl)-2-N-{4-[(2-tert-butylaminoethanoyl)methylamino]phenyl}-(2R,4R)-4-hydroxypyrrolidine-1,2-dicarboxamide,
1-N-(4-chlorophenyl)-2-N-{4-[(2-cyclopentylaminoethanoyl)-methylamino]phenyl}-(2R,4R)-4-hydroxypyrrolidine-1,2-dicarboxamide,
1-N-(4-chlorophenyl)-2-N-{4-[(2-cyclopropylmethylaminoethanoyl)methylamino]phenyl}-(2R,4R)-4-hydroxypyrrolidine-1,2-dicarboxamide,
1-N-(4-chlorophenyl)-2-N-{4-[(2-hydroxyethanoyl)methylamino]-phenyl}-(2R,4R)-4-hydroxypyrrolidine-1,2-dicarboxamide,
1-N-(4-chlorophenyl)-2-N-{4-[(2-methoxyethanoyl)methylamino]-phenyl}-(2R,4R)-4-hydroxypyrrolidine-1,2-dicarboxamide,
1-N-(4-chlorophenyl)-2-N-{4-[(2-ethoxyethanoyl)methylamino]-phenyl}-(2R,4R)-4-hydroxypyrrolidine-1,2-dicarboxamide,
1-N-(4-chlorophenyl)-2-N-{4-[(2-propoxyethanoyl)methylamino]phenyl}-(2R,4R)-4-hydroxypyrrolidine-1,2-dicarboxamide,
1-N-(4-chlorophenyl)-2-N-{4-[(2-butoxyethanoyl)methylamino]phenyl}-(2R,4R)-4-hydroxypyrrolidine-1,2-dicarboxamide,
1-N-(4-ethynylphenyl)-2-N-{4-[(2-methoxyethanoyl)methylamino]phenyl}-(2R,4R)-4-hydroxypyrrolidine-1,2-dicarboxamide,
1-N-(4-chlorophenyl)-2-N-{2-fluoro-4-[(2-methoxyethanoyl)-methylamino]phenyl}-(2R,4R)-4-hydroxypyrrolidine-1,2-dicarboxamide,
1-N-(4-chlorophenyl)-2-N-{5-[(2-methoxyethanoyl)methylamino]-pyridin-2-yl}-(2R,4R)-4-hydroxypyrrolidine-1,2-dicarboxamide,
and pharmaceutically usable solvates, salts and stereoisomers thereof, including mixtures thereof in all ratios.

2. Medicaments comprising at least one compound according to Claim 1 and/or pharmaceutically usable solvates and stereoisomers thereof, including mixtures thereof in all ratios, and optionally excipients and/or adjuvants.

3. Medicaments comprising at least one compound according to Claim 1 and/or pharmaceutically usable solvates and stereoisomers thereof, including mixtures thereof in all ratios, and at least one further medicament active ingredient.

4. Use of compounds according to Claim 1 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

5. Set (kit) consisting of separate packs of
(a) an effective amount of a compound according to Claim 1 and/or pharmaceutically usable solvates and stereoisomers thereof, including mixtures thereof in all ratios,
and
(b) an effective amount of a further medicament active ingredient.

6. Use of compounds according to Claim 1 and/or pharmaceutically usable solvates and stereoisomers thereof, including mixtures thereof in all ratios,
for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases, in combination with at least one further medicament active ingredient.

## Revendications

1. Composés choisis parmi le groupe
le 1-N-(4-chlorophényl)-2-N-{4-[(2-diméthylaminoéthanoyl)-méthylamino]phényl}-(2R,4R)-4-hydroxypyrrolidine-1,2-dicarboxamide,
le 1-N-(4-chlorophényl)-2-N-{4-[(2-(N-méthyl,N-butylamino)-éthanoyl)méthylamino]phényl}-(2R,4R)-4-hydroxypyrrolidine-1,2-dicarboxamide,
le 1-N-(4-chlorophényl)-2-N-{4-[(2-(morpholin-4-yl)éthanoyl)-méthylamino]phényl}-(2R,4R)-4-hydroxypyrrolidine-1,2-dicarboxamide,
le 1-N-(4-chlorophényl)-2-N-{4-[(2-(4-hydroxypipéridin-1-yl)-éthanoyl)méthylamino]phényl}-(2R,4R)-4-hydroxypyrrolidine-1,2-dicarboxamide,
le 1-N-(4-chlorophényl)-2-N-{4-[(2-(2,6-diméthylmorpholin-4-yl)-éthanoyl)méthylamino]phényl}-(2R,4R)-4-hydroxypyrrolidine-1,2-dicarboxamide,
le 1-N-(4-chlorophényl)-2-N-{4-[(2-(3-cyclohexylméthylpipéridin-1-yl)éthanoyl)méthylamino]phényl}-(2R,4R)-4-hydroxypyrrolidine-1,2-dicarboxamide,
le 1-N-(4-chlorophényl)-2-N-{4-[(2-diéthylaminoéthanoyl)méthyl-amino]phényl}-(2R,4R)-4-hydroxypyrrolidine-1,2-dicarboxamide,
le 1-N-(4-chlorophényl)-2-N-{4-[(2-(N-méthyl,N-éthylamino)-éthanoyl)méthylamino]phényl}-(2R,4R)-4-hydroxypyrrolidine-1,2-dicarboxamide,
le 1-N-(4-chlorophényl)-2-N-{4-[(2-(2-méthylimidazol-1-yl)-éthanoyl)méthylamino]phényl}-(2R,4R)-4-hydroxypyrrolidine-1,2-dicarboxamide,
le 1-N-(4-éthynylphényl)-2-N-{4-[(2-diméthylaminoéthanoyl)-méthylamino]phényl}-(2R,4R)-4-hydroxypyrrolidine-1,2-dicarboxamide,
le 1-N-(4-chlorophényl)-2-N-{2-fluoro-4-[(2-diméthylamino-éthanoyl)méthylamino]phényl}-(2R,4R)-4-hydroxypyrrolidine-1,2-dicarboxamide,
le 1-N-(4-chlorophényl)-2-N-{5-[(2-diméthylaminoéthanoyl)-méthylamino]pyridin-2-yl}-(2R,4R)-4-hydroxypyrrolidine-1,2-dicarboxamide,
le 1-N-(4-chlorophényl)-2-N-{4-[(2-acétoxyéthanoyl)méthylamino]phényl}-(2R,4R)-4-hydroxypyrrolidine-1,2-dicarboxamide,
le (2R,4R)-2-[({[4-({1-[1-(4-chlorophénylcarbamoyl)-4-hydroxypyrrolidin-2-yl]méthanoyl}amino)phényl]méthylcarbamoyl}méthyl)amino]-4-méthylpentanoate de méthyle,
le 1-N-(4-chlorophényl)-2-N-{4-[(2-éthylaminoéthanoyl)-méthylamino]phényl}-(2R,4R)-4-hydroxypyrrolidine-1,2-dicarboxamide,
le 1-N-(4-chlorophényl)-2-N-{4-[(2-chloroéthanoyl)méthylamino]phényl}-(2R,4R)-4-hydroxypyrrolidine-1,2-dicarboxamide,
le 1-N-(4-chlorophényl)-2-N-{4-[(2-cyclohexylaminoéthanoyl)-méthylamino]phényl}-(2R,4R)-4-hydroxypyrrolidine-1,2-dicarboxamide,
le 1-N-(4-chlorophényl)-2-N-{4-[(2-méthylaminoéthanoyl)méthylamino]phényl}-(2R,4R)-4-hydroxypyrrolidine-1,2-dicarboxamide,
le 1-N-(4-chlorophényl)-2-N-{4-[(2-isopropylaminoéthanoyl)-méthylamino]phényl}-(2R,4R)-4-hydroxypyrrolidine-1,2-dicarboxamide,
le 1-N-(4-chlorophényl)-2-N-{4-[(2-*tertio*-butylaminoéthanoyl)-méthylamino]phényl}-(2R,4R)-4-hydroxypyrrolidine-1,2-dicarboxamide,
le 1-N-(4-chlorophényl)-2-N-{4-[(2-cyclopentylaminoéthanoyl)-méthylamino]phényl}-(2R,4R)-4-hydroxypyrrolidine-1,2-dicarboxamide,
le 1-N-(4-chlorophényl)-2-N-{4-[(2-cyclopropylméthylamino-éthanoyl)méthylamino]phényl}-(2R,4R)-4-hydroxypyrrolidine-1,2-dicarboxamide,
le 1-N-(4-chlorophényl)-2-N-{4-[(2-hydroxyéthanoyl)méthylamino]phényl}-(2R,4R)-4-hydroxypyrrolidine-1,2-dicarboxamide,
le 1-N-(4-chlorophényl)-2-N-{4-[(2-méthoxyéthanoyl)méthylamino]phényl}-(2R,4R)-4-hydroxypyrrolidine-1,2-dicarboxamide,
le 1-N-(4-chlorophényl)-2-N-{4-[(2-éthoxyéthanoyl)méthylamino]phényl}-(2R,4R)-4-hydroxypyrrolidine-1,2-dicarboxamide,
le 1-N-(4-chlorophényl)-2-N-{4-[(2-propoxyéthanoyl)méthylamino]phényl}-(2R,4R)-4-hydroxypyrrolidine-1,2-dicarboxamide,
le 1-N-(4-chlorophényl)-2-N-{4-[(2-butoxyéthanoyl)méthylamino]phényl}-(2R,4R)-4-hydroxypyrrolidine-1,2-dicarboxamide,
le 1-N-(4-éthynylphényl)-2-N-{4-[(2-méthoxyéthanoyl)méthylamino]phényl}-(2R,4R)-4-hydroxypyrrolidine-1,2-dicarboxamide,
le 1-N-(4-chlorophényl)-2-N-{2-fluoro-4-[(2-méthoxyéthanoyl)-méthylamino]phényl}-(2R,4R)-4-hydroxypyrrolidine-1,2-dicarboxamide,
le 1-N-(4-chlorophényl)-2-N-{5-[(2-méthoxyéthanoyl)méthylamino]pyridin-2-yl}-(2R,4R)-4-hydroxypyrrolidine-1,2-dicarboxamide,
et les solvats, sels et stéréoisomères pharmaceutiquement utilisables de ceux-ci, y compris leurs mélanges en toutes proportions.

2. Médicaments comprenant au moins un composé selon la revendication 1 et/ou des solvats et stéréoisomères pharmaceutiquement utilisables de ceux-ci, y compris leurs mélanges en toutes proportions, et éventuellement des excipients et/ou des adjuvants.

3. Médicaments comprenant au moins un composé selon la revendication 1 et/ou des solvats et stéréoisomères pharmaceutiquement utilisables de ceux-ci, y compris leurs mélanges en toutes proportions, et au moins un autre ingrédient actif médicamenteux.

4. Utilisation de composés selon la revendication 1, et/ou de sels et solvats physiologiquement acceptables de ceux-ci, pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales.

5. Ensemble (kit) constitué de conditionnements séparés
(a) d'une quantité efficace d'un composé selon la revendication 1 et/ou de solvats et stéréoisomères pharmaceutiquement utilisables de ceux-ci, y compris leurs mélanges en toutes proportions,
et
(b) d'une quantité efficace d'un autre ingrédient actif médicamenteux.

6. Utilisation de composés selon la revendication 1 et/ou de solvats et stéréoisomères pharmaceutiquement utilisables de ceux-ci, y compris leurs mélanges en toutes proportions,
pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales, en association avec au moins un autre ingrédient actif médicamenteux.
